# FASCICULE DE BREVET EUROPEEN

(11) **EP 0 935 677 B1**
(45) Date de publication et mention de la délivrance du brevet: **27.03.2002**
(21) Numéro de dépôt: 97930585.1
(22) Date de dépôt: 25.06.1997
(51) Int. Cl.: C12Q 1/68, G01N 33/543

(54) **PROCEDE DE PREPARATION DE SUPPORT D'ANALYSE PRESENTANT UN ENSEMBLE ORDONNE DE ZONES DE REACTION**
VERFAHREN ZUR HERSTELLUNG EINES TESTTRÄGERS MIT GEORDNETEN REAKTIONSZONEN
METHOD FOR PREPARING ASSAY SUPPORTS HAVING AN ORDERED SET OF REACTION ZONES

(30) Priorité: 27.06.1996 FR 9608028
(43) Date de publication de la demande: 18.08.1999
(73) Titulaire: SARTORIUS AG, 37075 Göttingen (DE)
(72) Inventeur: DEMMER, Wolfgang, D-37077 Göttingen (DE); NUSSBAUMER, Dietmar, D-37079 Göttingen (DE); TO VINH, Khuong, D-31167 Bockenem (DE); NGUYEN, Thang, Thuong, F-45410 Vienne en Val (FR); BONFILS, Edwige, F-67200 Strasbourg (FR); DUPRET, Daniel, F-67500 Haguenau (FR)
(74) Mandataire: Breese, Pierre
(86) Numéro de dépôt international: FR9701132
(87) Numéro de publication internationale: WO9749834

(56) Documents cités:
- EP-A- 0 063 810
- WO-A-89/10977
- WO-A-94/12670
- WO-A-95/30774

## Description

La présente invention concerne le domaine de l'analyse de substances chimiques ou biologiques, comme des séquences d'acides nucléiques ou des protéines par réaction spécifique avec un ensemble de réactifs spécifiques fixés sur un support. Elle concerne plus particulièrement l'analyse d'acides nucléiques par hybridation avec des sondes oligonucléotidiques fixées sur un support. Ce type d'analyse trouve son application dans le séquençage, le diagnostic ou encore le contrôle qualité en recherche ou en production industrielle.

La loi d'appariement A-T (ou A-U) et G-C confère aux acides nucléiques la propriété de former des complexes d'hybridation spécifiques entre des séquences complémentaires. Cette propriété d'hybridation connue de longue date permet d'utiliser un fragment d'acide nucléique, ou un oligonucléotide, pour mettre en évidence la présence d'une séquence nucléique complémentaire. L'analyse de fragments d'ADN après séparation sur gel (E. Southern, *J. mol. Biol.,* 1975, 98, 503-517) est aujourd'hui largement répandue tant dans le domaine de la recherche fondamentale que dans celui de l'analyse médicale (Caskey, *Science*, 1987, 236, 1223-1228 ; Landegrer et al., *Science*, 1988, 242, 229-237 ; Arnheim et al., *Ann Rev. Biochem*., 1992, 61, 131-156).

Plusieurs techniques d'analyse des acides nucléiques basées sur leur propriété d'hybridation avec des sondes ont été développées notamment pour le séquençage d'ADN ou d'ARN inconnus, la détection de séquences associées à une pathologie, la recherche de mutations ponctuelles dans des séquences (S. Ikata et al., *Nuclei. Acids Res.,* 1987, 15, 797-811 ; J. A. Matthews, L. J. Krieka, *Analytical Biochemistry,* 1988, 169, 1-25).

Plus récemment, il a été proposé de nouveaux procédés d'analyse de fragments d'ADN inconnus, basés sur l'hybridation avec une série d'oligonucléotides comportant de 5 à 9 bases arrangées selon une séquence connue et immobilisés sur un support solide (E. Southern, Brevet Européen publié sour le numéro : 0 373 203 ; K. R. Khrapko et al., *FEBS Letters,* 1989, 256, 118-122 ; R. Drmanac et al., *Genomics*, 1989, 4, 114-128 ; R. Drmanac et al., *DNA and Cell Biology*, 1990, 9,527-534 ; K. R. Khrapko et al., *J. DNA Sequencing Mapp*., 1991, 1, 375-388 ; R. Drmanac et al., *Science,* 1993, 260, 1649-1652; R. J. Lipshutz, *J. Biomol. Struct. Dyn*., 1993, 11, 637-653 ; U. Maskos, E. Southern, *Nucleic Acids Res*., 1993, 21, 4663-4669 ; A. C. Pease et al., *Proc. Natl. Acad. Sci.,* USA, 1994, 91, 5022-5026 ; J. C. Williams, *Nucleic Acids Res.,* 1994, 22, 1365-1367 ; E. M. Southern, *Nucleic Acids Res.,* 1994, 22, 1368-1373).

Ces nouveaux procédés d'analyse sont fondés sur la préparation d'un support à la surface duquel est fixé une série de séquences oligonucléotidiques de même longueur couvrant l'ensemble des séquences possibles pour cette longueur, chacune des séquences oligonucléotidiques occupant des cellules, ou zones, séparées sur le support. La préparation et la mise en oeuvre d'un tel support est décrite notamment dans les demandes de brevet européen publiés sous les N° 373 203 et 392 546 ainsi pue dans la demande de brevet internationale publiée sous les N° WO 94/12670. La séquence d'acide nucléique à analyser est marquée et appliquée au support dans des conditions permettant l'hybridation avec les oligonucléotides, puis après lavage, on observe la localisation du marquage à la surface du support afin de détecter les signaux émis par les hybrides éventuellement formés entre le fragment d'ADN marqué analysé et un ou plusieurs oligonucléotides de la série immobilisés sur le support solide. Chaque position révélée positive sur le support correspond à une séquence complémentaire ainsi identifiée dans le fragment d'ADN analysé.

L'immobilisation des oligonucléotides à la surface du support peut être réalisée selon deux principes : soit une immobilisation après synthèse des oligonucléotides, soit la synthèse directe des oligonucléotides sur le support.

L'immobilisation après synthèse des oligonucléotides est difficile à mettre en oeuvre et ne semble pas suffisamment reproductible comme c'est le cas pour les anticorps (brevet européen N° 0 063 810), aussi, la plupart des équipes travaillant sur ce sujet, ont choisi de développer des techniques de synthèse directe sur le support qui sera utilisé pour l'hybridation et la détection (Demandes de brevet internationales WO 90 03 382, WO 90 15 070, WO 91 08 307, WO 91 07 087, WO 92 10 092, WO 92 10 587, WO 93 10 161, WO 93 09 668, WO 94 22 889, WO 95 11 748, WO 95 30 774). Celles-ci impliquent la synthèse sur une même surface d'une série de séquences oligonucléotidiques de même longueur couvrant toutes les séquences possibles pour cette longueur. Ainsi, pour des oligonucléotides de 2 bases, choisis parmi les quatre constituant l'ADN, 16 séquences sont possibles, pour des oligonucléotides de 3 bases, 64 séquences sont possibles, pour des oligonucléotides de 4 bases, 256 séquences sont possibles, pour des oligonucléotides de 5 bases, 1024 séquences sont possibles, pour des oligonucléotides de 6 bases, 4096 séquences sont possibles, etc... Or, plus la séquence des oligonucléotides est courte, moins elle est informative pour le séquençage; en effet, pour un fragment d'ADN de 1000 bases à séquencer, une séquence quelconque de 3 bases sera présente statistiquement au moins une quinzaine de fois et une séquence de 4 bases sera présente statistiquement probablement 4 fois, alors qu'une séquence de 5 bases ne devrait être présente statistiquement qu'une fois. En conséquence, ces techniques nécessitent la synthèse de plusieurs dizaines voir milliers d'oligonucléotides différents sur une même surface et la répétition de cette synthèse pour tous les supports préparés.

Or, il apparaît quasiment impossible de réaliser, selon les techniques de l'art antérieur, des dizaines de synthèses différentes sur un même support sans générer des erreurs, qui sont sources de positions non conformes sur chaque support. Il n'est donc pas possible de garantir l'exactitude de la séquence des différentes positions sur chaque support supposé identique. Dans ces conditions, la mise en oeuvre d'un contrôle de qualité qui permettrait de garantir totalement chaque position devient très difficile.

La présente invention vise précisément à fournir des supports à la surface desquels sont fixés dans des zones définies des réactifs spécifiques d'un type de réaction avec la substance analysée, et plus particulièrement des séquences oligonucléotidiques, dont les caractéristiques et les positions sont reproductibles et vérifiables, afin de réaliser aisément un controle de qualité.

Ce but est atteint grâce à un procédé de préparation de supports d'analyse de substances chimiques ou biologiques, présentant chacun un ensemble ordonné de zones de réaction où est susceptible de réagir ladite substance chimique ou biologique. Le procédé de l'invention est caractérisé en ce que l'on prépare N supports différents chacun homogène pour un type de réaction, puis en ce que l'on réuni entre-eux, selon un agencement prédéterminé, tout ou partie desdits N supports homogènes pour constituer des supports d'analyse présentant chacun un ensemble ordonné de zones de réaction d'au plus N types différents.

On entend par "type de réaction", la réaction spécifique susceptible de se produire entre la substance analysée et le support, lors de leur mise en contact. Cette réaction sera déterminée par la nature des N réactifs différents, présents de manière ordonnée et vérifiable sur les supports obtenus par le procédé de l'invention, chacun des N supports de départ étant homogène pour l'un de ses réactifs. Dans le cas de l'analyse d'acide nucléique, on entend par type de réaction, la réaction d'hybridation spécifique susceptible d'intervenir entre une séquence oligonucléotidique fixée sur l'un des N supports de départs, et que l'on retrouve dans une zone de réaction sur le support final, et une séquence d'acide nucléique complémentaire présente dans la substance analysée.

Selon une première forme particulière de mise en oeuvre, le procédé de l'invention comprend les étapes suivantes :
a) préparation de N supports homogènes pour un type de réaction de forme plate;
b) découpage desdits N supports homogènes plats en bandes de largeur sensiblement égale;
c) réunion sensiblement côte à côte des bandes provenant d'une partie au moins des N supports homogènes de façon à préparer des supports intermédiaires comportant selon un axe perpendiculaire auxdites bandes de supports homogènes, des alignements de zones de réaction différentes, chacun desdits alignements d'un même support intermédiaire étant identique;
d) découpage des supports intermédiaires en bandes de largeur sensiblement égale, chacune desdites bandes comprenant un desdits alignements de zones de réaction différentes,
e) réunion sensiblement côte à côte des bandes comprenant chacune un alignement de zones de réaction différentes de façon à constituer des supports d'analyse présentant chacun des alignements de zones de réaction ordonnées d'au plus N types différents.

Selon une seconde forme particulière de mise en oeuvre, le procédé de l'invention comprend les étapes suivantes :
a') préparation de N supports homogènes se présentant sous la forme de filaments ou de fibres, et
b') réunion sensiblement côte à côte de plusieurs desdits filaments de façon à préparer des supports intermédiaires comportant selon un axe perpendiculaire auxdits fils, des alignements de zones de réaction différentes, chacun desdits alignements d'un même support intermédiaire étant identique,
puis, réalisation des étapes (d) et (e) définies précédemment dans la première forme de mise en oeuvre.

L'étape (b') ci-dessus peut consister à coller côte à côte les filaments homogènes préparer à l'étape (a') de façon à former les supports intermédiaires définis à l'étape (c) du premier mode de réalisation.

Un variante des étapes (a) et (a') définies ci-dessus consite :
a") à préparer N supports homogènes se présentant sous la forme de filaments, et à procéder au tissage de chacun desdits N filaments et d'une maille constituée par un fil neutre du point de vue réactionnel, de façon à préparer les N supports homogènes du type de ceux de l'étape (a) définie dans le premier mode de réalisation, puis
à réaliser les étapes (b), (c), (d) et (e) définies précédemment.

Une variante des étapes (a") et (b') définies ci-dessus consiste à :
a') à préparer N supports homogènes se présentant sous la forme de filaments, et
b") à procéder au tissage de tout ou partie des N filaments homogène par entrelacement d'une trame formée desdits N filaments et d'une maille constituée par un fil neutre du point de vue réactionnel de façon à préparer des supports intermédiaires ou un support final, puis
à réaliser éventuellement les étapes (d) et (e) définies précédemment, si les supports préparés à l'étape (b") sont des supports intermédiaires.

Afin de maintenir le support intermédiaire des étapes (b), (b') et (b") rigide, celui-ci est avantageusement fixé sur un élément auxiliaire de soutien temporaire ou définitif; temporaire signifiant que l'élément de soutien auxiliaire sera ôté après l'étape (e) ou (b"), et définitif signifiant que l'élément de soutien auxiliaire sera laissé après l'étape (e) ou (b").

De même, le support final obtenu aux étapes (e) et (b") peut être avantageusement rigidifié et donc plus facilement manipulé en le fixant sur un élément de soutien. En outre, cet élément de soutien peut permettre de donner une forme particulière au support final, comme par exemple un ou plusieurs renfoncements facilitant et délimitant les dépôts des réactifs d'hybridation et de révélation au niveau de chaque zone de réaction, ou encore pour former un rebord au support.

Les supports homogènes sous forme plate ou de fibres peuvent être en une matière poreuse ou non poreuse. A titre de support plat poreux, on préfère une membrane microporeuse de cellulose.

Dans le cas où le support de l'invention est destiné à l'analyse d'acides nucléiques, les N supports de départ sont homogènes chacun pour un type de réaction d'hybridation spécifique. Avantageusement, chacun des N supports homogènes comporte alors une séquence oligonucluotidique différente. Selon une forme de réalisation particulière, destinée au séquençage ou à l'analyse de mutations, on fixe sur chacun des N supports homogènes une séquence oligonucluotidique différente mais de longueur égale, l'ensemble des séquences oligonicluotidiques des N supports couvrant alors l'ensemble des séquences possibles pour cette longueur.

L'analyse du degré d'hybridation avec chacun des oligonucléotides, par exemple par la valeur des Tm, au niveau de chaque zone de réaction des supports, permet de déterminer très précisément la séquence de l'acide nucléique, ADN, ou ARN analysé ou la présence d'une mutation.

Les supports de l'invention peuvent également être utilisés dans l'analyse immunologique; dans ce cas les N supports sont homogènes chacun pour un type de réaction antigène-anticorps. Dans ce mode de réalisation, un anticorps ou un antigène, selon que la substance analysée est un antigène ou un anticorps, différent sera fixé par toute méthode connue de l'homme du métier sur chacun des N supports homogènes de départ.

Les supports de l'invention peuvent également être utiliséspour l'analyse de substrats susceptible de réagir avec une série d'enzymes différentes placées chacune au niveau de chaque zone de réaction.

D'autres caractéristiques et avantages de l'invention apparaîtront à la lecture des exemples qui suivent, donnés à titre non limitatif.

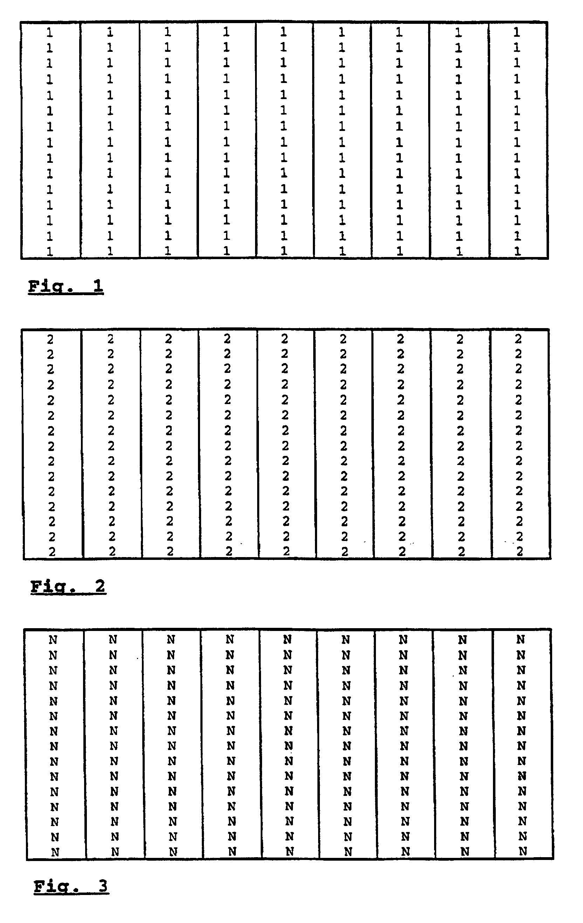

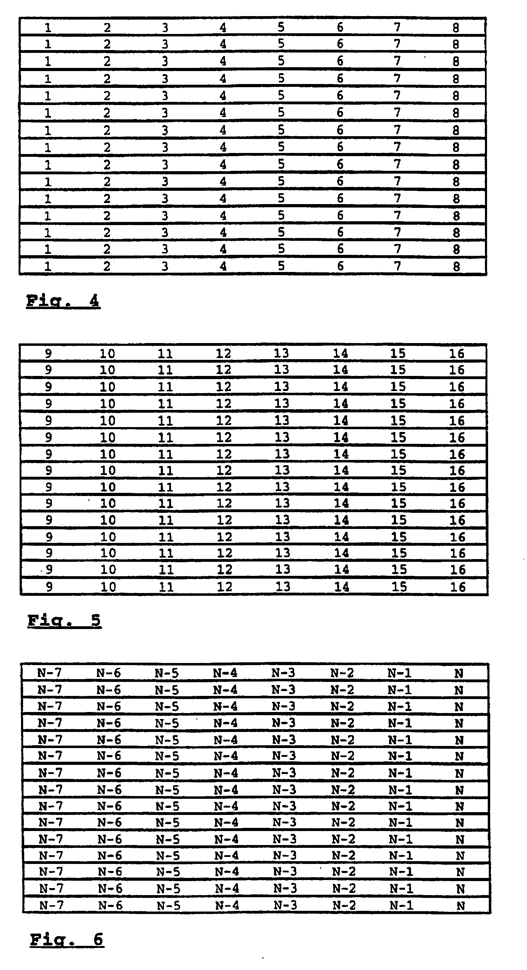

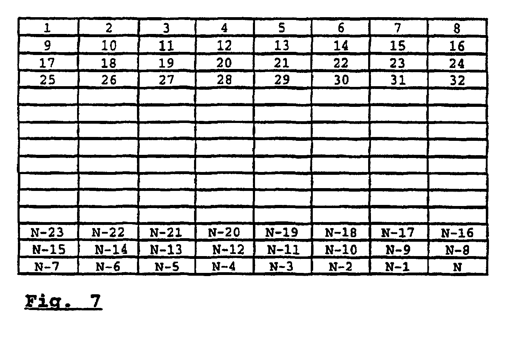

Un exemple de procédé de préparation d'un support d'analyse selon l'invention comprend les étapes (a) à (e) décrites ci-après et se référant aux figures 1 à 7 des dessins en annexe.
a) On prépare N supports homogènes pour un type de réaction. Par exemple en fixant sur chacun des N supports une séquence oligonucléotidique différente.
   Afin de faciliter la compréhension du procédé de l'invention, on a représenté dans les figures 1, 2 et 3, uniquement trois supports d'une série de N supports homogènes de forme plate :
   - le support de la figure 1, est homogène pour le type de réaction désigné (1) correspondant à une première séquence nucléique;
   - le support de la figure 2, est homogène pour le type de réaction désigné (2) correspondant à une deuxième séquence nucléique;
   - le support de la figure 3, est homogène pour le type de réaction désigné (N) correspondant à une nième séquence nucléique.
b) On découpe dans chacun des N supports homogènes, des bandes sensiblement de même largeur représentées dans les figures 1, 2 et 3 par les traits verticaux; ainsi, chacun des supports homogènes des figures 1 à 3 est découpé en 9 bandes.
c) A partir de ces bandes, on a préparé les supports intermédiaires des figures 4, 5 et 6, en plaçant côte à côte, sur un élément auxiliaire de soutien, des bandes provenant de l'étape (b). On voit parfaitement que chacun des supports intermédiaires comporte, selon les axes horizontaux, perpendiculaires aux bandes provenant des supports homogènes, des alignements de zones de réaction différentes, chacun desdits alignements d'un même support intermédiaire étant identique. On a représenté aux figures 4, 5 et 6, trois de ces supports intermédiaires, constitués chacun de 8 bandes préparées à l'étape (b) :
   - Le support de la figure 4, comporte 8 bandes verticales différentes portant chacune un même type de zone réaction, et 15 bandes horizontales indentiques comprenant chacune un alignement de 8 zones de réaction de type différent désignées: 1, 2 ,3 ,4, 5, 6, 7 et 8.
   - Le support de la figure 5, comporte un agencement identique à celui de la figure 4, mais avec alignement de 8 zones de réaction de type différent de ceux du support de la figure 4, désignées : 9, 10, 11, 12, 13, 14, 15 et 16.
   - Le support de la figure 6, représente le dernier support intermédiaire de la série de supports intermédiaires, sur lequel les bandes sont agencées comme sur les figures 4 et 5, avec un alignement de 8 zones de réaction différentes désignées : N-7, N-6, N-5, N-4, N-3, N-2, N-1 et N.
d) On a découpé les supports intermédiaires préparés à l'étape (c) en bandes, sensiblement de la même largeur, chacune desdites bandes comprenant un desdits alignements de zones de réaction différentes. On obtient ainsi à partir de chaque support intermédiaire un jeu de bandes identiques, chaque jeu étant différent. Le jeu de bandes issu du support intermédiaire de la figure 4, comprend 15 bandes de zones différentes désignées : 1, 2, 3, 4, 5, 6, 7 et 8 ; le jeu de bandes issu du support intermédiaire de la figure 5, comprend 15 bandes de zones différentes désignées : 9, 10, 11, 12, 13, 14, 15 et 16 ; le jeu de bandes issu du support intermédiaire de la figure 6, comprend 15 bandes de zones différentes désignées : N-7, N-6, N-5, N-4, N-3, N-2, N-1 et N.
e) On prépare les supports d'analyse en réunissant côte à côte, sur un élément de soutien final, une bande de chacun des jeux de bandes identiques préparés à l'étape (d) de façon à ce que chacun des supports d'analyse possède les N zones de réaction différentes. Dans l'exemple de la figure 7, on a représenté un des 15 supports d'analyse susceptibles d'être obtenus en utilisant toutes les bandes de l'étape (d).

### I - Préparation des N supports homogènes de l'étape (a).

### 1) La structure du support.

Les supports homogènes de l'étape (a) peuvent être poreux ou non poreux et de forme plate ou fibreux. A titre d'exemple, on peut citer les supports du tableau I ci-dessous.

**Tableau I**

| SUPPORTS | Plats | Fibreux |
|---|---|---|
| Non-poreux | feuilles, films | fils, grilles |
| Poreux | papier, nappes non-tissées, membranes de micro et ultrafiltration | mono-filaments, fils, multi-filaments, fibres creuses pour micro ou ultrafiltration |

Le choix d'un support poreux ou non poreux dépend de la densité de réactifs dont l'on souhaite disposer au niveau de chaque zone de réaction. Du fait de la plus grande surface disponible, les supports poreux offrent la possibilité d'atteindre une densité élevée de molécules.

Les supports poreux de forme plate préférés sont les membranes de micro-filtration dont la taille des pores est comprise entre 0,1 et 10 µm et l'épaisseur comprise entre 50 et 150 µm. Les fils constituent également un support poreux intéressant car ils combinent une surface importante et une forte stabilité mécanique, et leur épaisseur est comprise entre 50 et 500 µm.

### 2 - La matière constituant le support.

La matière constituant le support homogène dont la structure poreuse ou non, fibreuse ou plane, qui a été définie précédemment, est généralement un polymère organique, mais dans certains cas, comme les feuilles ou films, il peut s'agir également de métaux, par exemple d'aluminium. Si les éléments réactifs sont fixés chimiquement par des liaisons covalentes avec le support, tout polymère permettant ce type de liaison peut être utilisé. Parmi ceux-ci, on peut citer la cellulose, les polyamides, les polyuréthanes, les polystyrènes et polyacrylonitryles ou leur dérivé. Dans le cas des membranes ou fibres de micro-filtration, on préfère toutefois la cellulose, car ce matériau combine une bonne réactivité chimique avec une forte stabilité thermique et vis-à-vis les solvants organiques, ainsi qu'un fort caractère hydrophyle et une absorption non spécifique faible. Le caractère hydrophyle est en effet important pour l'humidification par des échantillons aqueux. La cellulose utilisée peut se présenter sous forme naturelle ou fibreuse, par exemple du type d'un filament de coton, mais aussi comme un produit régénéré, tel que des fibres de viscose. Les membranes de cellulose microporeuses sont dans tous les cas à base de cellulose régénérée, obtenue par saponification de membranes d'acétate de cellulose.

A l'inverse, une forte absorption du support est préjudiciable, si les éléments réactifs sont fixés sur le support par absorption. Ainsi, les antigènes et anticorps sont facilement absorbés sur le nitrate de cellulose. Les polyamides sont également intéressants, si l'absorption est utilisée pour fixer les éléments réactifs sur le support, de même que les polymères hydrophobes, comme les polyoléfines (polyéthylène et polypropylène), les polysulfones et polyethersulphones.

### 3 - Le type de réaction.

Les réactifs susceptibles de réagir avec la substance analysée au niveau de chaque zone de réaction sont fixés sur les supports selon les méthodes chimiques ou physiques connues dans l'art antérieur. Il peut s'agir de protéines antigéniques capables de réagir avec un anticorps ou à l'inverse d'anticorps capables de réagir avec une protéine ou partie de protéine. Il peut s'agir également, et c'est l'une des applications principales de l'invention, d'oligonucléotides sondes, qui sont fixés sur le support défini précédemment par toute méthode connue de l'homme du métier. Il peut s'agir d'une immobilisation après synthèse des oligonucléotides ou d'une synthèse directe des oligonucléotides sur le support, mettant en oeuvre des liaisons covalentes ou un bras de fixation qui peut supposer la préparation préalable des N supports. Une fixation physique peut également être réalisée, par absorption, échange d'ion, formation de chélate ou toute autre technique équivalente connue de l'homme du métier.

### II - La préparation des bandes et des supports intermédiaires des étapes (b) et (c).

### 1) Les bandes.

Si le support préparé à l'étape (a) est plat, celui-ci est coupé directement en bandes. Dans le cas de supports fibreux, ceux-ci sont avantageusement fixés sur un élément de soutien. Si N est un nombre important, il est préférable que la largeur des bandes soit la plus petite possible afin d'aboutir à des supports d'analyses de tailles réduites. Ainsi, une largeur de bande d'environ 0,1 mm semble raisonnable compte-tenu des moyens de découpe dont on dispose. Pour d'autres applications, dans lesquelles la taille du support d'analyse n'est pas critique, il est possible d'admettre des bandes d'une largeur d'environ 5mm.

### 2) Les supports intermédiaires.

Les bandes ou les fibres sont placées sur des éléments auxiliaires de soutien, soit côte à côte, soit directement au contact l'une de l'autre, soit en laissant un petit espace, de l'ordre de 0,1 à 1 mm, entre deux bandes, afin de faciliter le positionnement exact de chaque bande.

L'élément auxiliaire de soutien est une feuille ou un film plat en tout type de matière permettant de stabiliser les bandes constituant le support intermédiaire et le support final.

La mise en oeuvre de ces éléments de soutien auxiliaires peut n'être que temporaire, le support d'analyse final étant retiré de l'élément auxiliaire de soutien à la fin du procédé, ou, définitive, l'élément auxiliaire de soutien faisant alors partie intégrante du support d'analyse de l'invention. On préfère généralement, l'utilisation temporaire de ces éléments auxiliaires de soutien.

Dans le cas d'une utilisation définitive, la fixation des bandes sur les éléments de soutien auxiliaires doit être stable afin de ne pas interférer avec les conditions de l'analyse lors de la mise en oeuvre du support, par exemple, avec les conditions d'hybridation dans le cas ou les éléments réactifs des sites de réaction sont des oligonucléotides.

La fixation permanente des bandes sur l'élément de soutien auxiliaire peut être réalisée par des moyens très divers; il peut s'agir par exemple d'un laminage thermique, d'un soudage, d'un collage. Dans le cas de bandes préparées à partir de supports homogènes constitués de membranes microporeuses de cellulose, elles peuvent être fixées de façon définitive sur une nappe de polyoléfine (polypropylène, polyéthylène, ou des mélanges de ceux-ci) par un laminage thermique. Dans ce cas, la nappe est chauffée sous pression jusqu'à environ sa température de fusion, de façon à ce que le produit fondu pénètre les pores de la membrane et crée un lien mécanique homogène entre le support et l'élément de soutien auxiliaire après refroidissement. Une autre possibilité consiste à utiliser des adhésifs réactifs et des résines, notamment à base d'époxides, d'uréthanes ou d'acrylates. Généralement, les adhésifs non-réactifs ne présentent pas une stabilité d'adhésion suffisante à l'état humide.

La fixation temporaire des bandes sur l'élément de soutien auxiliaire peut être réalisée par laminage thermique à des températures et/ou pressions insuffisantes pour permettre une fixation permanente, ou encore avec des adhésifs présentant une faible adhésivité. A titre d'exemple, on peut utiliser une cassette adhésive du type de celle commercialisée par la Société Beiersdorf (Hamburg, Allemagne) sous la dénomination "Tesafilm".

### III - La découpe des supports intermédiaires de l'étape (d).

Les supports intermédiaires préparés à l'étape précédente sont constitués de bandes provenant de plusieurs supports homogènes initiaux fixées sur l'élément auxiliaire de soutien. La découpe de ces supports doit être réalisée avec une grande netteté et constitue de ce fait une phase critique. En effet, les bandes de supports homogènes initiaux et l'élément auxiliaire de soutien ne doivent pas être endommagés; en outre, la position des bandes de supports homogènes sur l'élément auxiliaire ne doit pas être modifiée pendant la découpe. La limite inférieure de la largeur des bandes découpées dans le support intermédiaire dépend des moyens de découpe et de maintien du support dont l'on dispose. Les moyens standards les plus sophistiqués, comme le Laser, permettent d'atteindre une largeur d'environ 0,1 mm, mais généralement une largeur de bandes supérieure à celles préparées à l'étape (b) peut être admise. Si par exemple, un fil d'une épaisseur de 50 µm est utilisé pour préparer le support homogène, une largeur de découpe à l'étape (d) de l'ordre de 1 à 2 mm permet de fixer un nombre important de molécules de réactif au niveau d'une zone de réaction. Toutefois, pour certaines applications où le nombre de molécules de réactif n'est pas critique, la largeur des bandes préparées aux étapes (b) et (d) peut être de l'ordre de plusieurs millimètres.

### IV - La préparation des supports d'analyse de l'étape (e).

Les supports d'analyse doivent être géométriquement stables à tous les stades d'application. Ils ne doivent pas se bomber ou rétrécir, par exemple lorsqu'ils sont soumis aux conditions d'hybridation, ils doivent demeurer suffisamment rigides pour être facilement manipulés. Les films polymères ou les nappes présentent ces propriétés; parmi ceux-ci, on peut citer à titre d'exemples : un film de polyéthylène-glycolterephtalate, d'une épaisseur de 50 à 500 µm et avantageusement de 100 à 250 µm ou des nappes de ployoléfine du même ordre d'épaisseur.

Si à l'étape (c), les bandes de supports homogènes de l'étape (a) ont été fixées sur des éléments auxiliaires de soutien de façon définitive, les supports d'analyse sont préparés en fixant les bandes préparées à l'étape (d) côte à côte sur un élément de soutien final; le coté des bandes qui est fixé sur l'élément de soutien final est celui correspondant à l'élément auxiliaire de soutien.

Si, à l'étape (c), les bandes du support intermédiaire ont été fixées de façon temporaire sur l'élément de soutien auxiliaire, les supports d'analyse sont préparés en fixant les bandes préparées à l'étape (d) côte à côte sur un élément de soutien final; le coté des bandes qui est fixé sur l'élément de soutien final est celui correspondant aux bandes de supports homogènes, l'élément de soutien auxiliaire étant retiré. La fixation définitive des bandes sur l'élément final de soutien est réalisée comme décrit à l'étape (c) pour la fixation permanente des bandes sur les éléments de soutien auxiliaires.

### V - Exemples détaillés.

### 1) Exemple n° 1.

a) On utilise N membranes microporeuses de cellulose régénérée du type SM 18606 commercialisées par la Société Sartorius AG (Göttingen, Allemagne) dont la taille des pores est de 0,45 µm. Sur chacune de ces N membranes, on réalise la synthèse directe ou on fixe un type de séquence oligonucléotidique, de façon à obtenir N membranes chacune homogène pour un type des N séquences oligonucléotidiques préalablement définies en fonction des séquences d'acides nucléiques que l'on souhaite analyser.
b) La synthèse des N oligonucléotides de séquences prédéfinies peut être réalisée avec un synthétiseur automatique en utilisant la méthode des phosphoramidites, soit directement sur les membranes définies ci-dessus en (a), soit sur un autre support de synthèse, et dans ce cas, en décrochant les oligonucléotides du support de synthèse lors de la dernière étape de déprotection, puis en fixant par toute fonction appropriée les oligonucléotides sur les membranes de cellulose définies en (a).
c) Des bandes de 2 mm de largeur sont découpées dans chacun des N supports homogènes préparés précédemment, puis les différentes bandes sont placées côte à côte sur des nappes d'un mélange de polypropylène et de polyéthylène de type FO 2432, FA Freudenberg, et soumises à un laminage à une température de 130-135°C sous une pression de 10N/cm² et une vitesse de 5m/min (longueur chauffée : 1,5 m).

Les supports intermédiaires ainsi préparés sont découpés à leur tour en bandes de 2 mm de largeur dans un sens perpendiculaire au sens de coupe des N supports homogènes de départ, puis déposés avec leur nappe sur une seconde nappe, laminés une seconde fois dans les mêmes conditions que précédemment.

### 2) Exemple n° 2.

Identique à l'exemple 1, mais à la dernière étape, les bandes sont collées avec un adhésif acrylate du type Macbond B2112, Fa. Mantac, d'un côté sur un film polymère, et de l'autre côté sur un film polyester du type BN 180, Fa. Kalle, Wiesbaden).

### 3) Exemple n°3.

N filaments de coton homogènes de 200 µm d'épaisseur portant chacun une séquence oligonucléotidique différente sont préparés comme à l'exemple 1 dans le cas d'une membrane de cellulose.

Différents filaments sont collés côte à côte sur des cassettes adhésives du type "Scotch Klebeband, ablösbar" de la Société 3M (USA), lesquelles sont découpées en bandes de 5 mm de largeur dans le sens perpendiculaire au filament. Les bandes ainsi préparées sont collées sur une cassette Macbond B 212 du côté des filaments alors que l'autre côté de la cassette Macbond est collé sur un film polyester comme à l'exemple 2, et les bandes de cassette Scotch sont retirées afin de rendre accessibles les morceaux de filaments.

## Revendications

1. Procédé de préparation de supports d'analyse de substances chimiques ou biologiques, lesdits supports présentant chacun un ensemble ordonné de zones de réaction où est susceptible de réagir ladite substance chimique ou biologique, **caractérisé en ce que** l'on prépare N supports différents chacun homogène pour un type de réaction, puis **en ce que** l'on réunit entre-eux, selon un agencement prédéterminé, tout ou partie desdits N supports homogènes pour constituer des supports d'analyse présentant chacun un ensemble ordonné de zones de réaction d'au plus N types différents.

2. Procédé de préparation de supports d'analyse de substances chimiques ou biologiques selon la revendication 1, **caractérisé en ce que** :
a) l'on prépare N supports homogènes de forme plate,
b) l'on découpe lesdits N supports homogènes plats en bandes de sensiblement la même largeur, puis
c) l'on réunit sensiblement côte à côte des bandes provenant d'une partie au moins des N supports homogènes de façon à préparer des supports intermédiaires comportant selon un axe perpendiculaire auxdites bandes de supports homogènes, des alignements de zones de réaction différentes, chacun desdits alignements d'un même support intermédiaire étant identique, puis
d) l'on découpe les supports intermédiaires en bandes, sensiblement de même largeur, chacune desdites bandes comprenant un desdits alignements de zones de réaction différentes, et
e) l'on réunit sensiblement côte à côte les bandes comprenant chacune un alignement de zones de réaction différentes de façon à constituer des supports d'analyse présentant chacun des alignements de zones de réaction ordonnées d'au plus N types différents.

3. Procédé de préparation de supports d'analyse de substances chimiques ou biologiques selon la revendication 1, **caractérisé en ce que** :
a') l'on prépare N supports homogènes se présentant sous la forme de filaments, et
b') l'on réunit sensiblement côte à côte plusieurs desdits filaments de façon à préparer des supports intermédiaires comportant selon un axe perpendiculaire auxdits fils, des alignements de zones de réaction différentes, chacun desdits alignements d'un même support intermédiaire étant identique, puis l'on réalise les étapes suivantes :
d) l'on découpe les supports intermédiaires en bandes, sensiblement de même largeur, chacune desdites bandes comprenant un desdits alignements de zones de réaction différentes, et
e) l'on réunit sensiblement côte à côte les bandes comprenant chacune un alignement de zones de réaction différentes de façon à constituer des supports d'analyse présentant chacun des alignements de zones de réaction ordonnées d'au plus N types différents.

4. Procédé de préparation de supports d'analyse de substances chimiques ou biologiques selon la revendication 1, **caractérisé en ce que** :
a") l'on prépare N supports homogènes se présentant sous la forme de filaments, et l'on procède au tissage de chacun desdits N filaments et d'une maille constituée par un fil neutre du point de vue réactionnel, de façon à préparer N supports homogènes de forme plate, puis l'on réalise les étapes suivantes :
b) l'on découpe lesdits N supports homogènes plats en bandes de sensiblement la même largeur, puis
c) l'on réunit sensiblement côte à côte des bandes provenant d'une partie au moins des N supports homogènes de façon à préparer des supports intermédiaires comportant selon un axe perpendiculaire auxdites bandes de supports homogènes, des alignements de zones de réaction différentes, chacun desdits alignements d'un même support intermédiaire étant identique, puis
d) l'on découpe les supports intermédiaires en bandes, sensiblement de même largeur, chacune desdites bandes comprenant un desdits alignements de zones de réaction différentes, et
e) l'on réunit sensiblement côte à côte les bandes comprenant chacune un alignement de zones de réaction différentes de façon à constituer des supports d'analyse présentant chacun des alignements de zones de réaction ordonnées d'au plus N types différents.

5. Procédé de préparation de supports d'analyse de substances chimiques ou biologiques selon la revendication 1, **caractérisé en ce que** :
a') l'on prépare N supports homogènes se présentant sous la forme de filaments, et
b") l'on procède au tissage de tout ou partie des N filaments homogène par entrelacement d'une trame formée desdits N filaments et d'une maille constituée par un fil neutre du point de vue réactionnel de façon à préparer des supports intermédiaires ou un support final, puis l'on réalise éventuellement les étapes suivantes :
d) l'on découpe les supports intermédiaires en bandes, sensiblement de même largeur, chacune desdites bandes comprenant un desdits alignements de zones de réaction différentes, et
e) l'on réunit sensiblement côte à côte les bandes comprenant chacune un alignement de zones de réaction différentes de façon à constituer des supports d'analyse présentant chacun des alignements de zones de réaction ordonnées d'au plus N types différents.

6. Procédé selon l'une quelconque des revendications 2-5, **caractérisé en ce qu'**aux étapes (b) et (b') et (b") le support intermédiaire est fixé sur un élément auxiliaire de soutien temporaire ou définitif.

7. Procédé selon l'une quelconque des revendications 2-6, **caractérisé en ce qu'**aux étapes (e) et (b") le support d'analyse est fixé sur un élément de soutien.

8. Procédé selon l'une quelconque des revendications précédentes, **caractérisé en ce que** les N supports homogènes sont en une matière poreuse ou non poreuse.

9. Procédé selon la revendication 8, **caractérisé en ce que** les N supports homogènes sont des membranes en cellulose.

10. Procédé de préparation de supports d'analyse d'acides nucléiques selon l'une quelconque des revendications précédentes, **caractérisé en ce que** les N supports sont homogènes chacun pour un type de réaction d'hybridation spécifique.

11. Procédé selon la revendication 10, **caractérisé en ce qu'**est fixée sur chacun des N supports homogènes une séquence oligonucluotidique différente.

12. Procédé selon la revendication 11, **caractérisé en ce qu'**est fixée sur chacun des N supports homogènes une séquence oligonucluotidique différente de longueur égale, l'ensemble des séquences oligonucluotidiques des N supports couvrant l'ensemble des séquences possibles pour cette longueur.

13. Procédé de préparation de supports d'analyse immunologique selon l'une quelconque des revendications 1 à 9, **caractérisé en ce que** les N supports sont homogènes chacun pour un type de réaction antigène-anticorps.

## Patentansprüche

1. Verfahren für die Herstellung von Substraten für die Analyse chemischer oder biologischer Substanzen, in dem die besagten Substrate jeweils eine geordnete Einheit von Reaktionszonen aufweisen, wo die besagte chemische oder biologische Substanz reagieren kann, **dadurch gekennzeichnet, dass** man N verschiedene jeweils homogene Substrate für einen Reaktionstyp vorbereitet, und dann dadurch, dass man nach einer vorgegebenen Anordnung die besagten N homogenen Substrate ganz oder teilweise miteinander verbindet, um Analysesubstrate zu bilden, die jeweils eine geordnete Einheit Reaktionszonen von höchstens N verschiedenen Typen aufweisen.

2. Verfahren für die Herstellung von Substraten für die Analyse chemischer oder biologischer Substanzen nach Anspruch 1, **dadurch gekennzeichnet, dass**:
a) man N homogene Substrate flacher Form herstellt,
b) man N homogene flache Substrate in ziemlich gleich breite Bänder zerschneidet
c) man dann Bänder, die mindestens von einem Teil der N homogenen Substrate stammen, nebeneinander anordnet, so dass Zwischensubstrate entstehen, die in einer zu den besagten Bändern homogener Substrate in rechtwinkliger Achse Reihen verschiedener Reaktionszonen enthalten, wobei alle besagten Reihen eines gleichen Zwischensubstrates identisch sind, und dann
d) man dann die Zwischensubstrate in ziemlich gleich breite Bänder zerschneidet, wobei jedes der besagten Bänder eine der besagten Reihen verschiedener Reaktionszonen enthält, und
e) die Bänder nebeneinander anordnet, die je eine Reihe verschiedener Reaktionszonen enthalten, um Analysesubstrate zu erhalten, die jeweils Reihen von geordneten Reaktionszonen von höchstens N verschiedenen Typen aufweisen.

3. Verfahren für die Herstellung von Substraten für die Analyse chemischer oder biologischer Substanzen nach Anspruch 1, **dadurch gekennzeichnet, dass**:
a') man N homogene Substrate herstellt, die die Form von Fäden aufweisen, und
b') man mehrere der besagten Fasern nebeneinander anordnet, um Zwischensubstrate zu erhalten, die jeweils in einer zu den besagten Fäden senkrechten Achse Reihen unterschiedlicher Reaktionszonen enthalten, wobei alle besagten Reihen eines gleichen Zwischensubstrates identisch sind, und dann folgende Schritte ausführt:
d) die Zwischensubstrate in ziemlich gleich breite Bänder zerschneidet, wobei jedes der besagten Bänder eine der besagten Reihen verschiedener Reaktionszonen enthält, und
e) die Bänder nebeneinander anordnet, die je eine Reihe verschiedener Reaktionszonen enthalten, um Analysesubstrate zu erhalten, die jeweils Reihen von geordneten Reaktionszonen von höchstens N verschiedenen Typen aufweisen.

4. Verfahren für die Herstellung von Substraten für die Analyse chemischer oder biologischer Substanzen nach Anspruch 1, **dadurch gekennzeichnet, dass**:
a") man N homogene Substrate herstellt, die die Form von Fäden aufweisen, und alle besagten N Fäden mit einer Masche aus einem im Hinblick auf die Reaktion neutralen Faden verwebt, so dass man N homogene Substrate flacher Form erhält, und dann die folgenden Schritte ausführt:
b) N homogene flache Substrate in ziemlich gleich breite Bänder zerschneidet und dann
c) Bänder, die mindestens von einem Teil der N homogenen Substrate stammen, nebeneinander anordnet, so dass Zwischensubstrate entstehen, die in einer zu den besagten Bändern homogener Substrate in rechtwinkliger Achse Reihen verschiedener Reaktionszonen enthalten, wobei alle besagten Reihen eines gleichen Zwischensubstrates identisch sind, und dann
d) die Zwischensubstrate in ziemlich gleich breite Bänder zerschneidet, wobei jedes der besagten Bänder eine der besagten Reihen verschiedener Reaktionszonen enthält, und
e) die Bänder nebeneinander anordnet, die je eine Reihe verschiedener Reaktionszonen enthalten, um Analysesubstrate zu erhalten, die jeweils Reihen von geordneten Reaktionszonen von höchstens N verschiedenen Typen aufweisen.

5. Verfahren für die Herstellung von Substraten für die Analyse chemischer oder biologischer Substanzen nach Anspruch 1, **dadurch gekennzeichnet, dass**:
a') man N homogene Substrate herstellt, die die Form von Fäden aufweisen, und
b") man die N homogenen Fäden ganz oder teilweise in einem Schuss verwebt, der aus den besagten N Fäden und einer Masche aus einem im Hinblick auf die Reaktion neutralen Faden besteht, und dann eventuell die folgenden Schritte ausführt:
d) man zerschneidet die Zwischensubstrate in ziemlich gleich breite Bänder, wobei jedes der besagten Bänder eine der besagten Reihen verschiedener Reaktionszonen enthält, und
e) man ordnet die Bänder nebeneinander an, die je eine Reihe verschiedener Reaktionszonen enthalten, um Analysesubstrate zu erhalten, die jeweils Reihen von geordneten Reaktionszonen von höchstens N verschiedenen Typen aufweisen.

6. Verfahren nach einem beliebigen der Ansprüche 2 - 5, **dadurch gekennzeichnet, dass** in den Schritten (b), (b') und (b") das Zwischensubstrat auf einem Hilfselement befestigt ist, das als vorläufige oder endgültige Stütze dient.

7. Verfahren nach einem beliebigen der Ansprüche 2 - 6, **dadurch gekennzeichnet, dass** in den Schritten (e) und (b") das Analysesubstrat auf einem stützenden Element befestigt ist.

8. Verfahren nach einem beliebigen der vorangehenden Ansprüche, **dadurch gekennzeichnet, dass** die N homogenen Substrate aus einer porösen oder nicht porösen Materie sind.

9. Verfahren nach Anspruch 8, **dadurch gekennzeichnet, dass** die N homogenen Substrate Membranen aus Zellstoff sind.

10. Verfahren für die Herstellung von Substraten für die Analyse von Nukleinsäuren nach einem beliebigen der vorangehenden Ansprüche, **dadurch gekennzeichnet, dass** die N Substrate jeweils für einen Typ einer spezifischen Hybridationsreaktion homogen sind.

11. Verfahren nach Anspruch 10, **dadurch gekennzeichnet, dass** auf jedem der N homogenen Substrate eine andere oligonukleotidische Sequenz fixiert ist.

12. Verfahren nach Anspruch 11, **dadurch gekennzeichnet, dass** auf jedem der N homogenen Substrate eine andere oligonukleotidische Sequenz gleicher Länge fixiert ist, wobei die Gesamtheit der oligonukleotidischen Sequenzen der N Substrate die Gesamtheit der für diese Länge möglichen Sequenzen abdecken.

13. Verfahren für die Herstellung immunologischer Analysesubstrate nach einem beliebigen der Ansprüche 1 bis 9, **dadurch gekennzeichnet, dass** die N Substrate jeweils für einen Reaktionstyp Antigen-Antikörper homogen sind.

## Claims

1. Method for preparing substrates for the assay of chemical or biological substances, said substrates each having an ordered assembly of reaction zones in which said chemical or biological substance is able to react, **characterized in that** N different substrates are prepared each one being homogeneous for one type of reaction, and **in that** between said substrates, according to a predetermined arrangement, all or part of said N homogeneous substrates are grouped together to form assay substrates each having an ordered assembly of reaction zones of no more than N different types.

2. Method for preparing assay substrates for chemical or biological substances according to claim 1, in which:
a) N homogeneous substrates of flat shape are prepared,
b) said N homogeneous flat substrates are cut into strips of substantially the same width, and
c) strips from at least one part of the N homogeneous substrates are grouped together substantially side by side so as to prepare intermediate substrates which, along an axis perpendicular to the said strips of homogeneous substrates, comprise alignments of different reaction zones, each of said alignments of one same intermediate substrate being identical, then
d) the intermediate substrates are cut into strips, of substantially the same width, each of said strips comprising one of said alignments of different reaction zones, and
e) the strips each comprising an alignment of different reaction zones are grouped together substantially side by side so as to form assay substrates each having alignments of ordered reaction zones of no more than N different types.

3. Method for preparing assay substrates for chemical or biological substances according to claim 1, **characterized in that**:
a') N homogeneous substrates are prepared in the form of filaments, and
b') several of said filaments are grouped together substantially side by side so as to prepare intermediate substrates which, along an axis perpendicular to said filaments, comprise alignments of different reaction zones, each of said alignments of one same intermediate substrate being identical, then the following steps are performed:
d) the intermediate substrates are cut into strips, of substantially the same width, each of said strips comprising one of said alignments of different reaction zones, and
e) the strips, each comprising an alignment of different reaction zones, are grouped together substantially side by side so as to form assay substrates each having alignments of ordered reaction zones of no more than N different types.

4. Method for preparing assay substrates for chemical or biological substances according to claim 1, **characterized in that**:
a") N homogeneous substrates are prepared in the form of filaments, and each of said N filaments is woven with a thread that is reactively neutral, so as to prepare N homogeneous substrates of flat shape, and subsequently the following steps are performed:
b) said N flat, homogenous substrates are cut into strips of substantially the same width, then,
c) the strips from at least part of the N homogeneous substrates are grouped substantially side by side so as to prepare intermediate substrates which, along an axis perpendicular to said strips of homogeneous substrates, comprise alignments of different reaction zones, each of said alignments from one same intermediate substrate being identical, then
d) the intermediate substrates are cut into strips, of substantially the same width, each of said strips comprising one of said alignments of different reaction zones, and
e) the strips, each comprising an alignment of different reaction zones, are grouped together substantially side by side so as to form assay substrates each having alignments of ordered reaction zones of no more than N different types.

5. Method for preparing assay substrates for chemical or biological substances according to claim 1, **characterized in that**:
a') N homogeneous substrates are prepared in the shape of filaments, and
b") all or part of the N homogeneous filaments are woven by weaving a weft formed of said N filaments with a warp formed of thread that is reactively neutral, so as to prepare intermediate substrates or an end substrate, then the following steps are optionally carried out:
d) the intermediate substrates are cut into strips, of substantially the same width, each of said strips comprising one of said alignments of different reaction zones, and
e) the strips each comprising an alignment of different reaction zones are grouped together substantially side by side so as to form assay substrates each having alignments of ordered reaction zones of no more than N different types.

6. Method according to any of claims 2 to 5, **characterized in that** at steps (b) (b') and(b") the intermediate substrate is fixed to a temporary auxiliary or a final support part.

7. Method according to any of claims 2 to 6, **characterized in that** at steps (e) and (b") the assay substrate is fixed to a supporting part.

8. Method according to any of the preceding claims, **characterized in that** the N homogenous substrates are in porous or non-porous material.

9. Method according to claim 8, **characterized in that** the N homogeneous substrates are cellulose membranes.

10. Method for preparing assay substrates for nucleic acids according to any one of the preceding claims, **characterized in that** the N substrates are each homogeneous for a specific type of hybridisation reaction.

11. Method according to claim 10, **characterized in that** a different oligonucleotide sequence is fixed to each of the N homogeneous substrates.

12. Method according to claim 11, **characterized in that** a different oligonucleotide sequence of equal length is fixed to each of the N homogeneous substrates, all the oligonucleotide sequences of the N substrates covering all the possible sequences for this length.

13. Method for preparing immunoassay substrates according to any of claims 1 to 9, **characterized in that** the N substrates are each homogeneous for a type of antigen-antibody reaction.
